(19) Europäisches Patentamt
European Patent Office
Office européen des brevets

(11) Veröffentlichungsnummer: **0 315 902 B1**

(12) **EUROPÄISCHE PATENTSCHRIFT**

(45) Veröffentlichungstag der Patentschrift: **18.03.92**

(51) Int. Cl.[5]: **C07D 209/12**, C07D 209/18, C07D 209/22, C07D 209/24

(21) Anmeldenummer: **88118361.0**

(22) Anmeldetag: **04.11.88**

# (54) Bis-indolyl-ethylen-aldehyde.

(30) Priorität: **11.11.87 DE 3738238**

(43) Veröffentlichungstag der Anmeldung:
**17.05.89 Patentblatt 89/20**

(45) Bekanntmachung des Hinweises auf die Patenterteilung:
**18.03.92 Patentblatt 92/12**

(84) Benannte Vertragsstaaten:
**CH DE FR GB LI**

(56) Entgegenhaltungen:
**FR-A- 2 259 883**

**JOURNAL OF HETEROCYCLIC CHEMISTRY, Band 22, Nr. 2, März-April 1985, Seiten 341-343, Hetero Corp., US; J. BERGMAN: "Reactions of indoles with ortho esters, N,N-dimenthylformamide and N,N-dimenthylacetamide dialkyl acetals"**

**LIEBIGS ANNALEN DER CHEMIE, Nr. 9, September 1988, Seiten 827-924, VCH Verlagsgesellschaft mbH, DE; C. FLO et al.: "Formylierung von Vinylindolen mit Diethoxycarbenium-tetrafluoroborat"**

(73) Patentinhaber: **BAYER AG**

**W-5090 Leverkusen 1 Bayerwerk(DE)**

(72) Erfinder: **Psaar, Hubertus, Dr.**
**Paul-Klee-Strasse 21**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft Bis-indolyl-ethylen-aldehyde der allgemeinen Formel

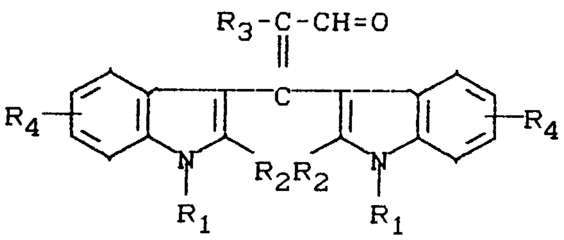

(I)

worin

- $R_1$ Wasserstoff, $C_1$- bis $C_{12}$-Alkyl, Benzyl, Phenylethyl oder Phenyl,
- $R_2$ Wasserstoff, $C_1$- bis $C_{12}$-Alkyl oder Phenyl,
- $R_3$ Wasserstoff, $C_1$- bis $C_{12}$-Alkyl, $C_2$- bis $C_{12}$-Alkenyl, COOH oder Phenyl und
- $R_4$ Wasserstoff, $C_1$- bis $C_{12}$-Alkyl, $C_1$- bis $C_{12}$-Alkoxy, Cyclohexoxy, Cyclopentoxy oder Halogen bedeuten, und

diese Substituenten ihrerseits gegebenenfalls durch Halogen, $C_1$- bis $C_4$-Alkoxy, Cyan oder eine Carboxylgruppe und Benzyl-, Phenylethyl-, Phenyl-, Cyclohexoxy-und Cyclopentoxyreste gegebenenfalls auch durch $C_1$- bis $C_4$-Alkylreste substituiert sind,
sowie ihre Herstellung.

Die Herstellung erfolgt durch Umsetzung von Bis-indolylethylenen der allgemeinen Formel

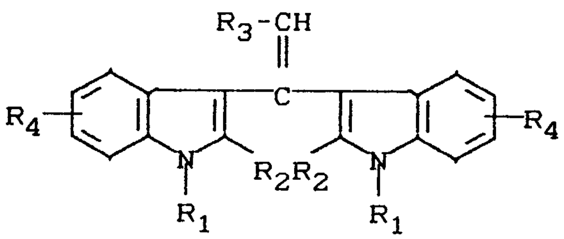

(II),

in der die verwendeten Symbole die bei Formel (I) angegebene Bedeutung haben,
in Phosphoroxychlorid mit Dimethylformamid oder durch Umsetzung von Indolen der allgemeinen Formel

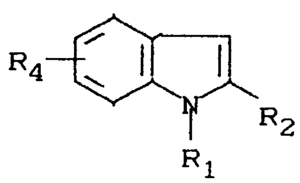

(III),

in der die verwendeten Symbole die bei Formel (I) angegebene Bedeutung haben,
mit Carbonsäuren der allgemeinen Formel

$R_3$-$CH_2$-COOH (IV),

in der $R_3$ die bei Formel (I) angegebne Bedeutung hat,
ihren Chloriden, Estern, beispielsweise Alkylestern, oder Anhydriden in Anwesenheit von Phosphoroxychlorid und ohne Isolierung Weiterreaktion mit Dimethylformamid.

Bevorzugt steht Halogen für Fluor, Chlor oder Brom, insbesondere Chlor.

Die bevorzugte Reaktionstemperatur der Umsetzung mit Dimethylformamid liegt bei 25 ° C bis 105 ° C, und die bevorzugte Reaktionszeit liegt zwischen 1 und 15 Stunden.

Dimethylformamid soll mindestens in molaren Mengen, bezogen auf die Ethylen-Verbindung zugesetzt werden.

Die Umsetzung kann mit oder ohne Lösungsmittel durchgeführt werden, wobei im letzteren Fall die Menge an Phosphoroxychlorid so bemessen sein soll, daß dieses gleichzeitig als Lösungsmittel wirkt. Als Lösungsmittel können Aromaten, alkylierte Aromaten oder chlorierte Aromaten eingesetzt werden, Geeignete Lösungsmittel sind z.B. Toluol, Xylol, Chlorbenzol oder Dichlorbenzol.

Das Molverhältnis von IV:III soll mindestens 1:2 betragen. Die bevorzugte Temperatur dieser Reaktion liegt bei 50 °C bis 105 °C.

Bevorzugt sind die Verbindungen der Formel I,
worin $R_2$ Phenyl ist und die übrigen Substituenten die obengenannte Bedeutung besitzen.

Besonders bevorzugt sind Aldehyde der Formel I, in der

- $R_1$ Wasserstoff, $C_1$- bis $C_{12}$-Alkyl oder Benzyl, das durch $C_1$- bis $C_4$-Alkyl, $C_1$- bis $C_4$-Alkoxy oder Halogen substituiert sein kann,
- $R_2$ Phenyl oder durch $C_1$- bis $C_4$-Alkyl, $C_1$- bis $C_4$-Alkoxy oder Halogen substituiertes Phenyl,
- $R_3$ Wasserstoff, $C_1$- bis $C_{12}$-Alkyl oder Phenyl und
- $R_4$ Wasserstoff, $C_1$- bis $C_{12}$-Alkyl, $C_1$- bis $C_{12}$-Alkoxy oder Halogen bedeuten,

und die Alkylreste gegebenenfalls durch Chlor, Cyan oder Carboxy substituiert sind.

Die Verbindungen der Formel I sind wertvolle Zwischenprodukte für Farbstoffe und Farbbildner, die beispielsweise in DE-A 3 738 240 beschrieben werden.

Beispiel 1

Zu 30 Gew.-Teilen Dimethylformamid werden 3,5 Gew.-Teile Phosphoroxychlorid gegeben und bei 50 °C 6,2 Gew.-Teile 1,1-Bis-(1,2-dimethyl-indolyl)-ethylen zugegeben. Der Ansatz wird 2 Stunden bei 50 °C gerührt, dann in 500 Gew.-Teile Eiswasser ausgetragen, mit Natronlauge 10 %ig alkalisch gestellt. Der Aldehyd wird abgesaugt und bei 50 °C im Vakuum getrocknet.
Ausbeute: 5,4 Gew.-Teil, F.: 208 °C.

Die Verbindung besitzt die Formel

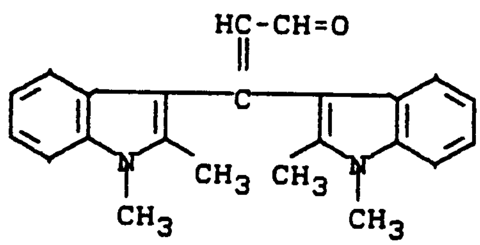

Beispiel 2

20,7 Gew.-Teile 1-Methyl-2-phenyl-indol werden in 30 ml Phosphoroxychlorid auf 80 bis 90 °C unter Rühren erhitzt. Bei dieser Temperatur werden 12 Gew.-Teile Essigsäureanhydrid zugetropft und der Ansatz 30 Minuten bei 100 °C gerührt. Dann werden 4,0 Gew.-Teile Dimethylformamid zugesetzt und der Ansatz weiter 1 Stunde bei 90 °C gerührt. Nach Abkühlen auf Raumtemperatur wird die Mischung in 700 Gew.-Teile Wasser gegossen und bis zur Kristallisation 20 Stunden gerührt. Der Aldehyd wird abgesaugt, mit Wasser gewaschen und bei 50 °C im Vakuum getrocknet. Die Verbindung wird aus Methanol unter Zusatz von Aktiv-Kohle umkristallisiert.
Die Ausbeute beträgt: 23,9 Gew.-Teile, F.: 227 °C.

Die Verbindung besitzt die Formel

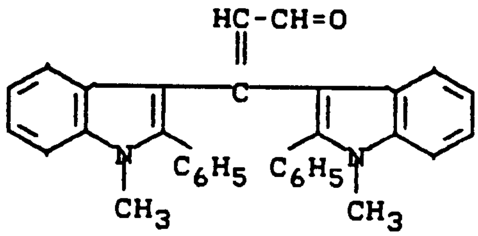

Beispiel 3

23,7 Gew.-Teile 1-Methyl-2-(4-methoxy-phenyl)-indol werden in 50 ml Phosphoroxychlorid auf 90 °C erhitzt und bei dieser Temperatur 3,7 Gew.-Teile Propionsäure zugetropft. Nach 30 Minuten wird auf Raumtemperatur abgekühlt und 100 ml Methanol langsam zugegeben. Anschließend wird dem Ansatz 3,8 Gew.-Teile Dimethylformamid zugesetzt und 1 Stunde bei 90 °C gerührt.
Der Ansatz wird in 500 Gew.-Teile Wasser gegossen, mit Natronlauge auf pH 10 gestellt. Das Produkt wird abgesaugt und bei 50 °C in Vakuum getrocknet.
Ausbeute: 24,2 Gew.-Teile, F.: 205 °C.

Die Verbindung der Formel

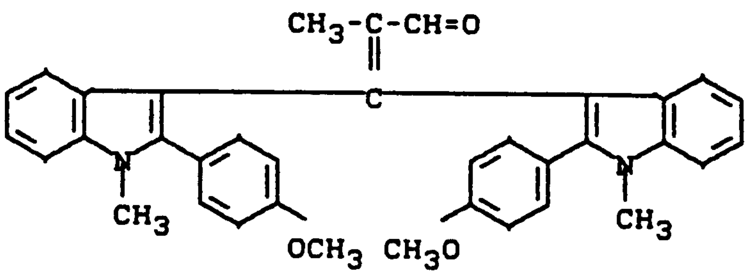

Gemäß Beispiel 1 bis 3 wurden folgene Aldehyde der Formel

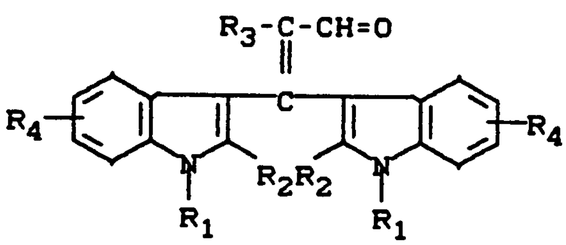

hergestellt:

| Beispiel | $R_1$ | $R_2$ | $R_3$ | $R_4$ | F. |
|---|---|---|---|---|---|
| 4 | $C_2H_4CN$ | $C_6H_5$ | H | H | 172 °C |
| 5 | H | $C_6H_5$ | H | H | 204 °C |
| 6 | $CH_3$ | $CH_3$ | H | H | 208 °C |
| 7 | $CH_3$ | H | H | H | 193-195 °C |
| 8 | $CH_3$ | $CH_3$ | $CH_3$ | H | 192 °C |
| 9 | $C_4H_9$ | $CH_3$ | H | H | Öl |
| 10 | $CH_3$ | $CH_3$ | $C_{10}H_{21}$ | H | Öl |
| 11 | $C_2H_4COOH$ | $C_6H_5$ | H | H | >300 °C |
| 12 | $C_2H_9$ | $C_6H_5$ | H | H | 114 °C |
| 13 | $C_6H_5CH_2$ | $C_6H_5$ | H | H | 195 °C |
| 14 | $C_6H_5CH_2$ | $CH_3$ | H | H | 145 °C |
| 15 | $CH_3$ | $C_6H_5$ | $CH_3$ | H | >325 °C |
| 16 | $CH_3$ | $C_6H_5$ | $C_2H_4Cl$ | H | 178 °C |
| 17 | $CH_3$ | $C_6H_4$,4-$OCH_3$ | H | H | 130 °C |
| 18 | $CH_3$ | $C_6H_4$,4-Cl | H | H | 213-215 °C |
| 19 | $CH_3$ | $C_6H_5$ | $CH_2=CH_2$ | H | 165-167 °C |
| 20 | $CH_3$ | $C_6H_5$ | H | 6-Cl | 229 °C |
| 21 | $CH_3$ | $C_6H_5$ | H | 5-$CH_3$ | 177 °C |
| 22 | $C_2H_5$ | $C_6H_5$ | H | H | 181-183 °C |
| 23 | $CH_3$ | $CH_2$-$CH(CH_3)_2$ | H | H | Öl |

## Patentansprüche

**1.** Bis-indolyl-ethylen-aldehyde der allgemeinen Formel

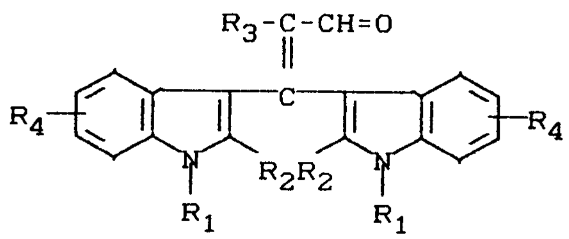

worin

- $R_1$ Wasserstoff, $C_1$- bis $C_{12}$-Alkyl, Benzyl, Phenylethyl oder Phenyl,
- $R_2$ Wasserstoff, $C_1$- bis $C_{12}$-Alkyl oder Phenyl,
- $R_3$ Wasserstoff, $C_1$- bis $C_{12}$-Alkyl, $C_2$- bis $C_{12}$-Alkenyl, COOH oder Phenyl und
- $R_4$ Wasserstoff, $C_1$- bis $C_{12}$-Alkyl, $C_1$- bis $C_{12}$-Alkoxy, Cyclohexoxy, Cyclopentoxy oder Halogen bedeuten, und

diese Substituenten ihrerseits gegebenenfalls durch Halogen, $C_1$- bis $C_4$-Alkoxy, Cyan oder eine Carboxylgruppe und Benzyl-, Phenylethyl-, Phenyl-, Cyclohexoxy-und Cyclopentoxyreste gegebenenfalls auch durch $C_1$- bis $C_4$-Alkylreste substituiert sind.

**2.** Bis-indolyl-ethylen-aldehyde gemäß Anspruch 1, dadurch gekennzeichnet, daß $R_2$ für Phenyl steht.

**3.** Bis-indolyl-ethylen-aldehyde gemäß Anspruch 1, dadurch gekennzeichnet, daß

- $R_1$ Wasserstoff, $C_1$- bis $C_{12}$-Alkyl oder Benzyl, das durch $C_1$- bis $C_4$-Alkyl, $C_1$- bis $C_4$-Alkoxy oder Halogen substituiert sein kann,
- $R_2$ Phenyl oder durch $C_1$- bis $C_4$-Alkyl, $C_1$- bis $C_4$-Alkoxy oder Halogen substituiertes Phenyl,
- $R_3$ Wasserstoff, $C_1$- bis $C_{12}$-Alkyl oder Phenyl und
- $R_4$ Wasserstoff, $C_1$- bis $C_{12}$-Alkyl, $C_1$- bis $C_{12}$-Alkoxy oder Halogen bedeuten,

und die Alkylreste gegebenenfalls durch Chlor, Cyan oder Carboxy substituiert sind.

**4.** Verfahren zur Herstellung von Bis-indolyl-ethylenaldehyden des Anspruchs 1, dadurch gekennzeichnet, daß man Bis-indolyl-ethylene der allgemeinen Formel

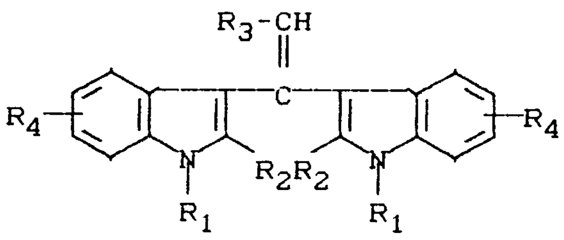

,

in der die verwendeten Symbole die in Anspruch 1 angegebene Bedeutung haben, in Phosphoroxychlorid mit Dimethylformamid umsetzt.

**5.** Verfahren zur Herstellung von Bis-indolyl-ethylenaldehyden des Anspruchs 1, dadurch gekennzeichnet, daß man Indole der allgemeinen Formel

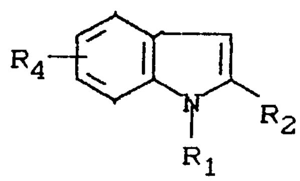

,

in der die verwendeten Symbole die in Anspruch 1 angegebene Bedeutung haben, mit Carbonsäuren der allgemeinen Formel

$R_3$-$CH_2$-COOH,

in der $R_3$ die in Anspruch 1 angegebene Bedeutung hat, ihren Chloriden, Estern oder Anhydriden in Anwesenheit von Phosphoroxychlorid umsetzt und anschließend ohne Isolierung mit Dimethylformamid umsetzt.

**Claims**

**1.** Bis(indolyl)ethylene aldehydes of the general formula

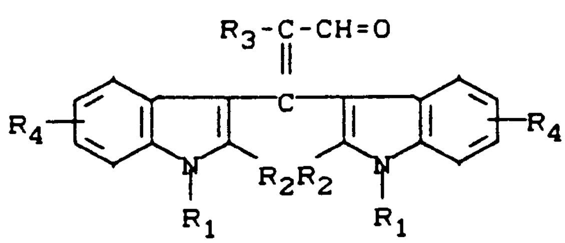

in which

- $R_1$ denotes hydrogen, $C_1$- to $C_{12}$-alkyl, benzyl, phenylethyl or phenyl,
- $R_2$ denotes hydrogen, $C_1$- to $C_{12}$-alkyl or phenyl,
- $R_3$ denotes hydrogen, $C_1$- to $C_{12}$-alkyl, $C_2$- to $C_{12}$-alkenyl, COOH or phenyl and
- $R_4$ denotes hydrogen, $C_1$- to $C_{12}$-alkyl, $C_1$- to $C_{12}$-alkoxy, cyclohexoxy, cyclopentoxy or halogen, and

these substituents themselves are optionally substituted by halogen, $C_1$- to $C_4$-alkoxy, cyano or a

carboxyl group, and benzyl, phenylethyl, phenyl, cyclohexoxy and cyclopentoxy radicals optionally also by $C_1$- to $C_4$-alkyl radicals.

**2.** Bis(indolyl)ethylene aldehydes according to Claim 1, characterised in that $R_2$ represents phenyl.

**3.** Bis(indolyl)ethylene aldehydes according to Claim 1, characterised in that

$R_1$ denotes hydrogen, $C_1$- to $C_{12}$-alkyl or benzyl, which can be substituted by $C_1$- to $C_4$-alkyl, $C_1$– to $C_4$-alkoxy or halogen,

$R_2$ denotes phenyl or phenyl substituted by $C_1$- to $C_4$-alkyl, $C_1$- to $C_4$-alkoxy or halogen,

$R_3$ denotes hydrogen, $C_1$- to $C_{12}$-alkyl or phenyl and

$R_4$ denotes hydrogen, $C_1$- to $C_{12}$-alkyl, $C_1$- to $C_{12}$-alkoxy or halogen,

and the alkyl radicals are optionally substituted by chlorine, cyano or carboxyl.

**4.** Process for the preparation of bis(indolyl)ethylene aldehydes of Claim 1, characterised in that bis-(indolyl)ethylenes of the general formula

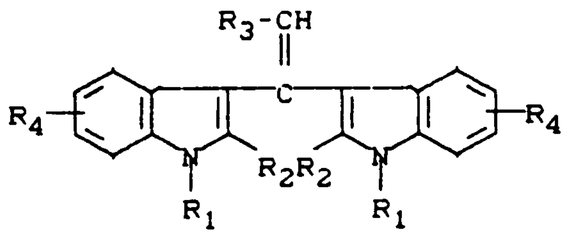

,

in which the symbols used have the meaning given in Claim 1,
are reacted in phosphorus oxychloride with dimethylformamide.

**5.** Process for the preparation of bis(indolyl)ethylene aldehydes of Claim 1, characterised in that indoles of the general formula

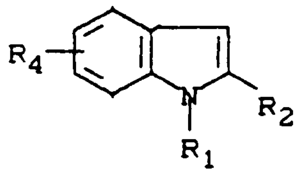

,

in which the symbols used have the meaning given in Claim 1,
are reacted with carboxylic acids of the general formula

$R_3$-$CH_2$-COOH,

in which $R_3$ has the meaning given in Claim 1,
their chlorides, esters or anhydrides in the presence of phosphorus oxychloride and are then reacted, without isolation, with dimethylformamide.

## Revendications

**1.** Aldéhydes bis-indolyl-éthyléniques de formule générale

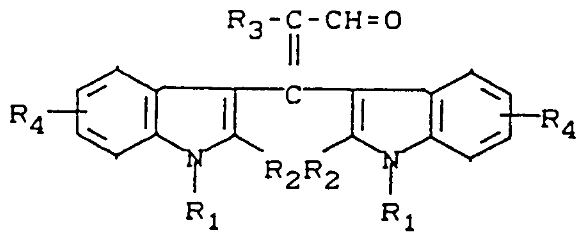

dans laquelle
$R_1$ représente un atome d'hydrogène, un groupe alkyle en $C_1-C_{12}$, benzyle, phényléthyle ou phényle,
$R_2$ représente un atome d'hydrogène, un groupe alkyle en $C_1$-$C_{12}$ ou phényle,
$R_3$ représente un atome d'hydrogène, un groupe alkyle en $C_1$-$C_{12}$, alcényle en $C_2$-$C_{12}$, COOH ou phényle, et
$R_4$ représente un atome d'hydrogène, un groupe alkyle en $C_1$-$C_{12}$, alcoxy en $C_1$-$C_{12}$, cyclohexoxy, cyclopentoxy ou un atome d'halogène, et
ces substituants peuvent pour leur part être éventuellement substitués par de l'halogène, par des restes alcoxy en $C_1$-$C_4$, cyano ou par un groupe carboxyle, et les restes benzyle, phényléthyle, phényle, cyclohexoxy et cyclopentoxy sont éventuellement aussi substitués par des restes alkyles en $C_1$-$C_4$.

**2.** Aldéhydes bis-indolyl-éthyléniques selon la revendication 1, caractérisés en ce que $R_2$ représente un groupe phényle.

**3.** Aldéhydes bis-indolyl-éthyléniques selon la revendication 1, caractérisé en ce que :
$R_1$ représente un atome d'hydrogène, un groupe alkyle en $C_1$-$C_{12}$ ou benzyle, qui peut être substitué par un reste alkyle en $C_1$-$C_4$, alcoxy en $C_1$-$C_4$, alcoxy en $C_1$-$C_4$ ou par de l'halogène,
$R_2$ représente un groupe phényle ou bien un groupe phényle substitué par un reste alkyle en $C_1$-$C_4$, alcoxy en $C_1$-$C_4$ ou par de l'halogène,
$R_3$ représente un atome d'hydrogène, un groupe alkyle en $C_1$-$C_{12}$ ou phényle, et
$R_4$ repésente un atome d'hydrogène, un groupe alkyle en $C_1$-$C_{12}$, alcoxy en $C_1$-$C_{12}$ ou de l'halogène,
et les restes alkyles sont éventuellement substitués par du chlore, par un reste cyano carboxy.

**4.** Procédé pour préparer des aldéhydes bis-indolyl-éthyléniques selon la revendication 1, caractérisé en ce qu'on fait réagir des bis-indolyl-éthylène de formule générale

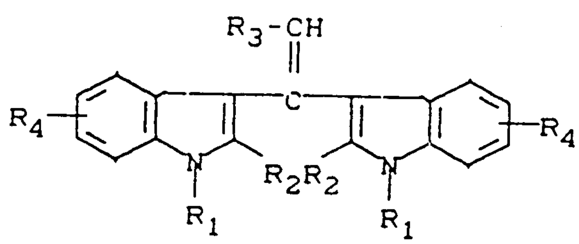 ,

dans laquelle les symboles utilisés ont le sens indiqué à la revendication 1,
dans de l'oxychlorure de phosphore avec du diméthylformamide.

**5.** Procédé pour préparer des aldéhydes bis-indolyl-éthyléniques selon la revendication 1, caractérisé en ce qu'on fait réagir des indoles de formule générale

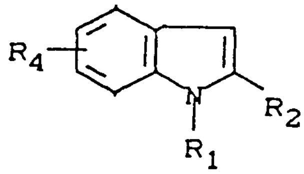

,

dans laquelle les symboles utilisés ont le sens indiqué à la revendication 1, avec des acides carboxyliques de formule générale

$R_3$-$CH_2$-COOH,

dans laquelle $R_3$ a le sens indiqué à la revendication 1, avec leurs chlorures, esters ou anhydrides en présence d'oxychlorure de phosphore et l'on fait ensuite réagir, sans isolement, avec du diméthylformamide.